# FASCICULE DE BREVET EUROPEEN

(11) **EP 2 866 109 B1**
(45) Date de publication et mention de la délivrance du brevet: **06.05.2020**
(21) Numéro de dépôt: 14188939.4
(22) Date de dépôt: 15.10.2014
(51) Int. Cl.: G05B 19/12, G05B 19/42, B24B 9/14, G02C 7/02, G06T 1/00

(54) **Procédé et dispositif d'acquisition et de calcul de données d'un objet ophtalmique**
Verfahren und Vorrichtung zur Erfassung und Berechnung von Daten eines ophthalmischen Gegenstands
Method and device for acquiring and calculating data of an ophthalmic object

(30) Priorité: 25.10.2013 FR 1360446
(43) Date de publication de la demande: 29.04.2015
(73) Titulaire: LUNEAU TECHNOLOGY OPERATIONS, 27340 Pont de l'Arche (FR)
(72) Inventeur: Videcoq, Jean-Jacques, 76570 PAVILLY (FR); Vassard, Michaël, 76520 BOOS (FR)
(74) Mandataire: Lavoix

(56) Documents cités:
- FR-A1- 2 854 268
- FR-A1- 2 959 831
- US-A- 5 604 583
- US-A1- 2013 075 465
- US-B2- 6 837 580

## Description

La présente invention est relative à un procédé d'acquisition et de calcul de données géométriques d'au moins un motif associé à un objet ophtalmique, tel qu'un verre de présentation, une lentille ophtalmique ou un gabarit, pour la fabrication de lentilles ophtalmiques semblables à l'objet ou complémentaires, procédé du type dans lequel on utilise un dispositif d'acquisition et de calcul de données géométriques qui comprend :
- un support transparent adapté pour porter un objet ophtalmique ;
- d'un côté du support, des moyens d'éclairage de ce support ;
- de l'autre côté du support, une caméra vidéo orientée vers le support et adaptée pour produire un signal vidéo représentatif d'au moins un motif associé à l'objet ophtalmique posé sur le support ; et
- des moyens d'analyse et de traitement de signal recevant en entrée le signal vidéo produit par la caméra, et adaptés pour calculer et fournir des données géométriques destinées à la réalisation de la lentille.

Un verre de présentation est un verre en matière plastique transparente, non correctif, ayant le contour, et éventuellement au moins un perçage, analogues à ceux d'une lentille (ou verre) ophtalmique à réaliser. Un gabarit est un objet plan ayant la même forme que le verre à reproduire. Il peut être muni de perçages à reproduire.

Un motif géométrique désignera généralement le contour de l'objet, ou des lignes de marquage repérant notamment l'axe géométrique d'orientation d'une lentille ophtalmique, mais il pourra également désigner des perçages de fixation formés dans un verre de présentation, dans une lentille ou dans un gabarit représentant une lentille finie. D'autres lignes de marquage peuvent constituer des motifs, notamment des lignes de marquage du centre de la pupille d'un utilisateur.

Dans les procédés ci-dessus, les motifs sont toujours des motifs utilisés pour la fabrication des lentilles, c'est-à-dire pour le pilotage d'une machine d'usinage, notamment de meulage, des lentilles à partir d'une ébauche circulaire.

En particulier, le FR-A-2 854 268 décrit un procédé du type précité permettant de positionner avec précision un adaptateur sur une ébauche de verre optique et d'obtenir des données utilisées pour le pilotage d'une machine de meulage et/ou de perçage de verres ophtalmiques à commande numérique.

Dans tous les cas, l'objet ophtalmique à analyser doit être positionné sur le support transparent dans une position prédéterminée, en ce qui concerne tant la face de l'objet qui se trouve en regard du support que son orientation dans son plan général. Une erreur sur le verre lui-même ou sur son positionnement conduit à la mise au rebut de la lentille.

L'invention a pour but de permettre de tenir compte de façon facile et fiable de l'objet ophtalmique réellement utilisé ainsi que de son positionnement sur le support.

A cet effet, l'invention a pour objet un procédé du type précité, caractérisé en ce que :
(a) on trace sur l'objet ophtalmique un motif de vérification indépendant desdites données géométriques, ce motif de vérification étant asymétrique par rapport à chacun de deux axes perpendiculaires l'un à l'autre ;
(b) on positionne l'objet ophtalmique sur le support transparent du dispositif d'acquisition et d'affichage ; et
(c) au moyen dudit dispositif, on saisit optiquement et on analyse ledit motif de vérification.

Suivant des modes de mise en oeuvre de ce procédé :
- à partir de l'analyse dudit motif de vérification, on détermine les calculs à réaliser sur lesdites données géométriques ;
- le dispositif comporte un moniteur comportant un écran, et on affiche sur l'écran du moniteur les verres de la monture en ayant replacé automatiquement les verres à leurs positions respectives droite ou gauche, et en ayant réorienté chaque verre par rapport à un axe de monture associé à la monture ;
- on affiche en outre sur l'écran du moniteur une alerte fonction de la nature de l'objet ophtalmique, de la face de l'objet dirigée vers l'écran et/ou de l'orientation de cet objet autour d'un axe perpendiculaire au support ;
- ledit dispositif contient des informations représentatives d'images-étalons dudit motif de vérification pour chacun des objets ophtalmiques et pour chacun des modes de positionnement possibles de ces objets ophtalmiques, et l'étape (c) est réalisée au moyen dudit dispositif de la manière suivante :
   - (c1) correction de l'orientation de l'image de l'objet ophtalmique.
   - (c2) reconnaissance optique dudit motif de vérification saisi ; et
   - (c3) comparaison du motif de vérification saisi avec lesdites images-étalons
- à l'étape (a), on trace également sur l'objet une indication représentative d'une caractéristique de la monture, notamment la mesure du pont, et cette indication est reconnue par lecture optique et utilisée par ledit dispositif pour le calcul des données destinées à la réalisation de la lentille.

De façon correspondante, l'invention a également pour objet un dispositif d'acquisition et d'affichage de données géométriques d'au moins un motif associé à un objet ophtalmique, tel qu'un verre de présentation, une lentille ou un gabarit, pour la fabrication de lentilles ophtalmiques semblables à l'objet ou complémentaires, du type comprenant :
- un support transparent adapté pour porter un objet ophtalmique ;
- d'un côté du support, des moyens d'éclairage de ce support ;
- de l'autre côté du support, une caméra vidéo orientée vers le support et adaptée pour produire un signal vidéo représentatif d'au moins un motif associé à l'objet ophtalmique posé sur le support ; et
- des moyens d'analyse et de traitement de signal recevant en entrée le signal vidéo produit par la caméra, et adaptés pour calculer et fournir des données géométriques destinées à la réalisation de la lentille ;
le dispositif étant caractérisé en ce qu'il comprend des moyens de reconnaissance optique et d'analyse de l'image réelle d'un motif de vérification indépendant desdites données géométriques porté par l'objet ophtalmique positionné sur le support transparent, ce motif de vérification étant asymétrique par rapport à chacun de deux axes perpendiculaires l'un à l'autre.

Suivant des modes de réalisation de ce dispositif :
- il comprend des moyens de détermination des calculs à réaliser sur lesdites données géométriques à partir de l'analyse dudit motif de vérification ;
- il comprend un moniteur comportant un écran et des moyens pour afficher sur l'écran les verres de la monture en ayant replacé automatiquement les verres à leurs positions respectives droite ou gauche, et en ayant réorienté chaque verre par rapport à un axe de monture associé à la monture ;
- il comprend des moyens d'affichage sur l'écran d'une alerte fonction de l'objet ophtalmique, de la face de cet objet dirigée vers l'écran et/ou de l'orientation de cet objet autour d'un axe perpendiculaire au support ;
- lesdits moyens d'affichage comportent des moyens d'affichage d'une fenêtre dans laquelle sont indiquées la nature de l'objet, ladite face et/ou ladite orientation ;
- il comprend des moyens de stockage d'informations représentatives d'images-étalons, pour au moins un objet ophtalmique, d'un motif de vérification asymétrique par rapport à chacun de deux axes perpendiculaires l'un à l'autre, ces images-étalons correspondant à chaque objet ophtalmique et aux différents positionnements possibles de cet objet ophtalmique sur le support avec une orientation prédéterminée de l'objet ophtalmique, des moyens de correction de l'orientation de l'image de l'objet ophtalmique posé sur le support, et des moyens de comparaison de ladite image réelle et desdites images-étalons.

Des exemples de réalisation de l'invention vont maintenant être décrits en regard des dessins annexés, sur lesquels :
- la figure 1 représente schématiquement, de face, une monture équipée de deux verres de présentation adaptés pour la mise en oeuvre du procédé suivant l'invention ;
- la figure 2 représente schématiquement un dispositif d'acquisition et d'affichage conforme à l'invention ;
- la figure 3 représente schématiquement une partie du dispositif de la figure 2 ;
- les figures 4 à 7 représentent diverses images d'un verre de présentation de la monture de la figure 1, telles qu'affichées sur l'écran du dispositif de la figure 2, en fonction du positionnement de ce verre sur le support du dispositif ;
- la figure 8 est une variante de la figure 6 ; et
- la figure 9 représente schématiquement une variante du dispositif de la figure 2.

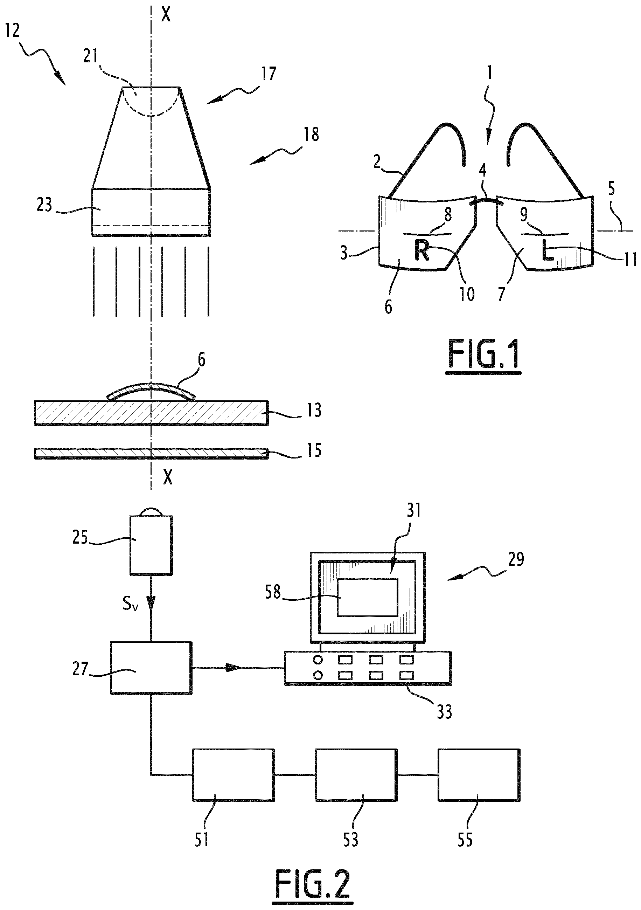

On a représenté sur la figure 1 une monture de lunettes 1 qui comprend deux branches 2 et deux cercles de monture 3 reliés par un pont 4. A cette monture 1 est associé un axe de monture 5 parallèle à la tangente aux deux cercles 3 au sommet de ceux-ci. La monture est supposée symétrique par rapport à un plan vertical médian.

La monture 1 est équipée de deux verres de présentation droit 6 et gauche 7, transparents et non correctifs, dont la forme doit être reproduite avec précision à partir d'une ébauche de verre optique afin de constituer une paire de lunettes.

Chaque verre 6, 7 porte sur l'une de ses faces un trait 8, 9 qui s'étend suivant l'axe de monture 5. Pour cette raison, on désigne le trait 8, 9 par l'expression « axe de monture du verre ».

Chaque verre 6, 7 porte en outre un motif de vérification respectif 10, 11 qui est indépendant des données optiques et géométriques utilisées pour piloter la machine d'usinage, par exemple de meulage. Chaque motif de vérification 10, 11 est propre au verre 6 ou 7. De plus, il est asymétrique par rapport à toute droite parallèle à l'axe de monture 5 et par rapport à toute droite perpendiculaire à cet axe. De préférence, chaque motif de vérification 10, 11 est chiral, c'est-à-dire qu'il ne présente aucun axe de symétrie compris dans son plan.

Dans l'exemple représenté, le motif de vérification 10 du verre de présentation droit 6 est constitué par la lettre R, tandis que le motif de vérification 11 du verre de présentation gauche 7 est constitué par la lettre L.

Ces deux motifs de vérification sont tracés à la main sur la face convexe (face avant) des deux verres 6, 7, sensiblement parallèlement au trait 8, 9.

Sur la figure 2, on a représenté schématiquement un dispositif 12 d'acquisition, de mesure et d'affichage conforme à l'invention, qui peut notamment, pour l'essentiel, être tel que décrit dans le FR-A-2 854 268 précité, avec un mode d'analyse et de calcul analogue.

Ce dispositif comprend une plaque transparente en verre 13, plane et horizontale, constituant un support pour l'objet ophtalmique à analyser. Celui-ci est ici constitué par le verre de présentation 6 droit, qui repose par ses bords périphériques sur la surface plane supérieure de la plaque de verre 13. Le verre 6 a donc sa face concave tournée vers le support 13. Sous la plaque-support 13 est disposé, parallèlement, un écran de projection plan 15, qui peut être notamment constitué d'une plaque de verre dépoli ou d'une feuille de matériau translucide, du type calque. Des moyens 17 d'éclairage de l'objet sont disposés au-dessus de la plaque-support 13, de façon à éclairer la totalité de l'objet 6 et à projeter une ombre de l'objet sur l'écran de projection 15, au travers de la plaque support 13. Ces moyens d'éclairage 17 sont essentiellement constitués d'une source lumineuse 21, par exemple une LED, et d'un ensemble optique ou collimateur 23, en général formé d'un ensemble de lentilles. Cet ensemble 23 est destiné à canaliser le rayonnement lumineux émis par la source, et à assurer un éclairage régulier de l'objet 6 avec des rayons lumineux verticaux.

L'image de l'objet 6 formée sur l'écran de projection 15, cette image étant en fait l'ombre de l'objet sur cet écran 15, est observée par une caméra vidéo matricielle 25 reliée à une unité d'analyse d'image et de traitement de signal 27, elle-même reliée à un moniteur 29. Le moniteur 29 comprend un écran d'affichage 31 et un pavé 33 de commande et de réglage de l'affichage.

L'unité d'analyse d'image 27 comporte une entrée connectée à la sortie de la caméra 25 pour recevoir le signal vidéo Sᵥ produit par la caméra, et une sortie connectée au moniteur 29 d'une manière décrite plus loin.

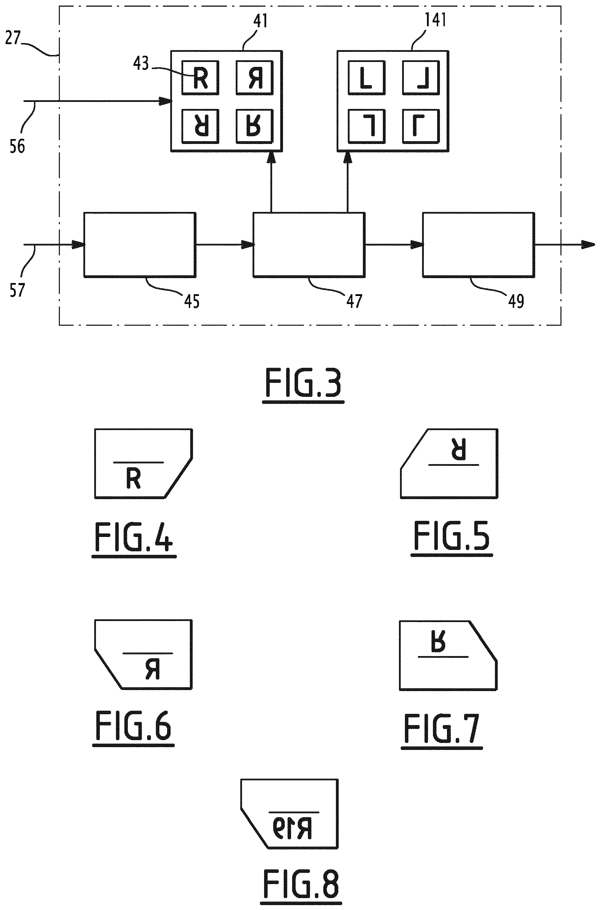

Comme schématisé à la figure 3, l'unité 27 comporte une mémoire 41 contenant les images-étalons 43 du motif 10 dans les quatre cas possibles de positionnement du verre 6 sur le support 13, représentées sur les figures 4 à 7 :
- pose face concave et position droite (figure 4),
- pose face concave et position inversée (figure 5),
- pose face convexe et position droite (figure 6),
- pose face convexe et position inversée (figure 7).

Les expressions « position droite » et « position inversée » définissent l'orientation du verre 6 autour d'un axe vertical, c'est-à-dire perpendiculaire au support 13.

Ces positions s'entendent avec l'axe de monture disposé horizontalement.

L'unité 27 comporte également un dispositif 45 de reconnaissance optique de caractères, un comparateur 47 relié au dispositif 45 et à la mémoire 43, et un dispositif 49 relié au comparateur 47.

Bien entendu, l'unité 27 comporte une seconde mémoire 141 analogue à la mémoire 41 et relative au motif 11. Le comparateur 47 est également relié à cette seconde mémoire.

Le dispositif comprend en outre des moyens de programmation 51 reliés d'une part à l'unité d'analyse et de traitement 27 et d'autre part à une unité de commande 53 de la meuleuse 55.

Comme décrit dans le FR-A-2 854 268 précité, l'unité d'analyse et de traitement 27 peut comprendre des moyens de correction d'image pour tenir compte de la distorsion des pixels en fonction de la distance à l'axe central vertical X-X de la caméra 25 et des moyens d'éclairage 21. Elle comprend dans tous les cas des moyens de correction de l'orientation de l'axe de monture 8, 9 porté par l'objet posé sur le support 13.

Dans ce qui suit, on ne décrira que la partie du procédé d'analyse et d'affichage relative au motif de vérification 10 ou 11, étant entendu que l'analyse et l'affichage du contour du verre 6, 7 et de l'axe de monture 8, 9 qu'il porte s'effectuent de façon classique, par exemple de la manière décrite dans le FR-A précité.

L'opérateur pose l'un des deux verres sur le support 13, avec son axe de monture sensiblement horizontal.

L'image du verre est tout d'abord redressée ou « ré-axée » de façon que l'axe de monture 8, 9 soit horizontal. Pour cela, l'unité de traitement d'image 27 identifie l'axe de monture 8, 9 et opère une rotation de l'image afin que cet axe de monture soit horizontal. Ce ré-axage de la monture est décrit dans le FR-A précité

Puis l'image du verre captée par la caméra 25 est transmise au dispositif 45 par une ligne 57 indiquée sur la figure 3. L'image du motif de vérification, localisée dans l'image par le dispositif 45, est comparée en 47 aux images-étalons des mémoires 41 et 141 et identifiée.

Ainsi, le dispositif 12 reconnaît s'il s'agit du verre droit ou gauche, et de quelle manière il est positionné. Il adapte lui-même ses calculs au verre 6 ou 7 présent sur le support, et au positionnement de celui-ci, pour générer les données géométriques nécessaires à la meuleuse.

Ainsi :
- si on se trouve dans la configuration de la Fig. 6, l'inversion du motif de vérification selon un axe vertical permet de déterminer que le verre droit a été posé sur sa face convexe en position droite, et le dispositif associe cette position au contour du verre, pour la suite du traitement.
- si on se trouve dans la configuration de la Fig. 7, l'inversion du motif selon les deux axes horizontal et vertical permet de déterminer que le verre droit a été posé sur sa face convexe, mais que le verre est tourné de 180 °par rapport à la position qu'il a dans la monture. L'unité de traitement réalise automatiquement une rotation de 180° du contour et associe au contour obtenu la face convexe du verre ;
- si on se trouve dans la configuration de la Fig. 4 ou 5, la non inversion ou l'inversion selon l'axe vertical permet de déterminer que le verre a été posé sur sa face concave. Comme cette configuration peut mener à des imprécisions sur la mesure du contour, suivant la courbure et la forme du verre, un message peut, dans une variante, être envoyé à l'utilisateur, via le dispositif 49 et l'écran 31, pour l'informer. L'opérateur peut alors soit continuer le traitement dans cette configuration, soit recommencer la saisie de l'image après avoir placé le verre sur sa face convexe.

De plus, le dispositif 49 fait afficher sur l'écran 31 une fenêtre 58 (figure 2) dans laquelle les deux verres de la monture sont dessinés en affichant le verre droit et le verre gauche à leurs places respectives.

Pour cela, le contour du verre a été préalablement ré-axé par rapport à l'axe de monture 8, 9. A cet effet, l'unité de traitement d'image 27 identifie l'axe de monture 8, 9 et opère une rotation de l'image afin que cet axe de monture soit horizontal. Ce réaxage de la monture est décrit dans le FR-A précité.

Comme on le comprend, le motif de vérification 10, 11 est analysé en même temps et de la même manière que le contour et le trait 8, et éventuellement que d'autres motifs portés par le verre de présentation et utilisés pour le meulage.

Eventuellement, le motif de vérification peut être complété par une indication manuscrite sensiblement horizontale représentative de la dimension du pont 4 de la monture, et/ou d'une autre donnée utilisée pour la réalisation des lentilles ophtalmiques. L'unité 27 est alors adaptée pour reconnaître cette donnée et pour l'afficher sur l'écran 31 et/ou en tenir compte sans que l'opérateur ait besoin de l'entrer manuellement.

Ainsi, dans l'exemple représenté sur la figure 8, la lettre R est associée au nombre 19, représentatif de la largeur (en mm) du pont 4.

Ceci permet éventuellement à l'opérateur d'effectuer une vérification supplémentaire.

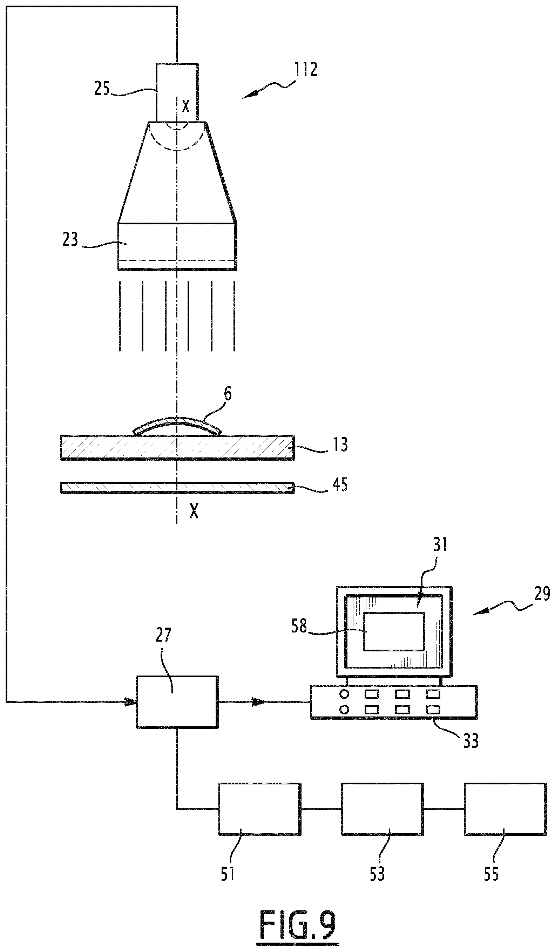

Le dispositif d'acquisition et d'affichage 112 représenté à la figure 9 ne diffère du dispositif 12 de la figure 2 que par l'interversion de la position des moyens d'éclairage et de la caméra 25.

En effet, les moyens d'éclairage sont constitués par un diffuseur plan et horizontal 45 disposé sous le support 13, à la place de l'écran 15 de la figure 2, tandis que la caméra 25 est disposée au-dessus du collimateur 23, en regardant vers le bas suivant l'axe X-X. Dans ce cas, c'est l'image de l'objet posé sur le support 13 qui est captée par la caméra, et non plus son ombre sur un écran comme c'est le cas avec le dispositif de la figure 2.

Le dispositif 112 peut être utilisé de manière analogue au dispositif 12 pour effectuer la vérification de la nature (droite ou gauche) et du positionnement du verre de présentation utilisé. Bien entendu, les images de vérification sont alors inversées droite-gauche pour chacune des quatre situations de positionnement.

Dans tous les cas, les données géométriques calculées par le dispositif 12 ou 112 sont utilisées d'une part pour positionner un adaptateur de montage sur une ébauche de la lentille à réaliser, puis pour piloter la meuleuse 55. La première de ces étapes, dite de blocage, peut être effectuée dans le dispositif 12 ou 112, ou dans un dispositif de blocage séparé auquel les données calculées sont fournies.

## Revendications

1. Procédé d'acquisition et de calcul de données géométriques d'au moins un motif associé à un objet ophtalmique (6, 7), tel qu'un verre de présentation, une lentille ophtalmique ou un gabarit, pour la fabrication de lentilles ophtalmiques semblables à l'objet ou complémentaires, procédé du type dans lequel on utilise un dispositif (12 ; 112) d'acquisition et de calcul de données géométriques qui comprend :
- un support transparent (13) adapté pour porter un objet ophtalmique ;
- d'un côté du support, des moyens (17) d'éclairage de ce support ;
- de l'autre côté du support, une caméra vidéo (25) orientée vers le support et adaptée pour produire un signal vidéo représentatif d'au moins un motif associé à l'objet ophtalmique posé sur le support ; et
- des moyens (27) d'analyse et de traitement de signal recevant en entrée le signal vidéo produit par la caméra, et adaptés pour calculer et fournir des données géométriques destinées à la réalisation de la lentille,
le procédé étant **caractérisé en ce que** :
(a) on trace sur l'objet ophtalmique (6, 7) un motif de vérification (10, 11) indépendant desdites données géométriques, ce motif de vérification étant asymétrique par rapport à chacun de deux axes perpendiculaires l'un à l'autre ;
(b) on positionne l'objet ophtalmique sur le support transparent (13) du dispositif d'acquisition et d'affichage (12, 112) ;
(c) au moyen dudit dispositif (12, 112), on saisit optiquement et on analyse ledit motif de vérification ; et
(d) à partir de l'analyse du motif de vérification, on détermine la face de l'objet ophtalmique (6, 7) sur laquelle repose l'objet ophtalmique (6, 7) positionné sur le support (13),
ledit dispositif (12; 112) contenant des informations représentatives d'images-étalons dudit motif de vérification (10, 11) pour chacun des objets ophtalmiques (6, 7) et pour chacun des modes de positionnement possibles de ces objets ophtalmiques, et **en ce que** l'étape (c) est réalisée au moyen dudit dispositif de la manière suivante :
- (c1) correction de l'orientation de l'image de l'objet ophtalmique.
- (c2) reconnaissance optique dudit motif de vérification saisi ; et
- (c3) comparaison du motif de vérification saisi avec lesdites images-étalons.

2. Procédé suivant la revendication 1, **caractérisé en ce que**, à partir de l'analyse dudit motif de vérification, on détermine les calculs à réaliser sur lesdites données géométriques.

3. Procédé suivant la revendication 1 ou 2, **caractérisé en ce que** le dispositif comporte un moniteur (29) comportant un écran (31), et **en ce qu'**on affiche sur l'écran (31) du moniteur (29) les verres de la monture (6,7) en ayant replacé automatiquement les verres à leurs positions respectives droite ou gauche, et en ayant réorienté chaque verre par rapport à un axe de monture (5) associé à la monture.

4. Procédé suivant la revendication 3, **caractérisé en ce qu'**on affiche en outre sur l'écran (31) du moniteur (29) une alerte fonction de la nature de l'objet ophtalmique (6, 7), de la face de l'objet dirigée vers l'écran et/ou de l'orientation de cet objet autour d'un axe perpendiculaire au support.

5. Procédé suivant l'une quelconque des revendications 1 à 4, **caractérisé en ce que**, à l'étape (a), on trace également sur l'objet (6, 7) une indication représentative d'une caractéristique de la monture, notamment la mesure du pont (4), et **en ce que** cette indication est reconnue par lecture optique et utilisée par ledit dispositif pour le calcul des données destinées à la réalisation de la lentille.

6. Procédé suivant l'une quelconque des revendications précédentes, **caractérisé en ce que** le motif de vérification est chiral.

7. Dispositif d'acquisition et d'affichage de données géométriques d'au moins un motif associé à un objet ophtalmique (6, 7), tel qu'un verre de présentation, une lentille ou un gabarit, pour la fabrication de lentilles ophtalmiques semblables à l'objet ou complémentaires, du type comprenant :
- un support transparent (13) adapté pour porter un objet ophtalmique ;
- d'un côté du support, des moyens (17, 45) d'éclairage de ce support ;
- de l'autre côté du support, une caméra vidéo (25) orientée vers le support et adaptée pour produire un signal vidéo représentatif d'au moins un motif associé à l'objet ophtalmique posé sur le support ; et
- des moyens (27) d'analyse et de traitement de signal recevant en entrée le signal vidéo produit par la caméra, et adaptés pour calculer et fournir des données géométriques destinées à la réalisation de la lentille ;
le dispositif étant **caractérisé en ce qu'**il comprend
- des moyens (45) de reconnaissance optique et d'analyse de l'image réelle d'un motif de vérification (10, 11) indépendant desdites données géométriques porté par l'objet ophtalmique (6, 7) positionné sur le support transparent (13), ce motif de vérification étant asymétrique par rapport à chacun de deux axes perpendiculaires l'un à l'autre,
- des moyens de détermination, à partir de l'analyse de l'image réelle dudit motif de vérification (10, 11), de la face de l'objet ophtalmique (6, 7) sur laquelle repose l'objet ophtalmique (6, 7) positionné sur le support (13),
- des moyens (41 ; 141) de stockage d'informations représentatives d'images-étalons, pour au moins un objet ophtalmique (6, 7), d'un motif de vérification (10, 11) asymétrique par rapport à chacun de deux axes perpendiculaires l'un à l'autre, ces images-étalons correspondant à chaque objet ophtalmique et aux différents positionnements possibles de cet objet ophtalmique sur le support avec une orientation prédéterminée de l'objet ophtalmique ;
- des moyens de correction de l'orientation de l'image de l'objet ophtalmique (8, 9) posé sur le support ; et
- des moyens (47) de comparaison de ladite image réelle et desdites images-étalons.

8. Dispositif suivant la revendication 7, **caractérisé en ce qu'**il comprend des moyens de détermination des calculs à réaliser sur lesdites données géométriques à partir de l'analyse dudit motif de vérification.

9. Dispositif suivant la revendication 8, **caractérisé en ce qu'**il comprend :
- un moniteur (29) comportant un écran (31) ; et
- des moyens pour afficher sur l'écran (31) les verres de la monture (6,7) en ayant replacé automatiquement les verres à leurs positions respectives droite ou gauche, et en ayant réorienté chaque verre par rapport à un axe de monture (5) associé à la monture.

10. Dispositif suivant la revendication 9, **caractérisé en ce qu'**il comprend des moyens (49) d'affichage sur l'écran d'une alerte (58) fonction de l'objet ophtalmique, de la face de cet objet dirigée vers l'écran et/ou de l'orientation de cet objet autour d'un axe perpendiculaire au support.

11. Dispositif suivant la revendication 10, **caractérisé en ce que** lesdits moyens d'affichage (49) comportent des moyens d'affichage d'une fenêtre dans laquelle sont indiquées la nature de l'objet, ladite face et/ou ladite orientation.

12. Dispositif suivant l'une quelconque des revendications 7 à 11, **caractérisé en ce que** le motif de vérification (10, 11) est chiral.

## Patentansprüche

1. Verfahren zum Beschaffen und Berechnen von geometrischen Daten von mindestens einem Muster, das einem ophthalmischen Objekt (6, 7), wie einem Präsentationsglas, einer Augenlinse oder einer Schablone, zugeordnet ist, für die Herstellung von ophthalmischen Linsen ähnlich dem Objekt oder komplementär dazu, wobei das Verfahren von einer Art ist, bei der eine Vorrichtung (12; 112) zum Beschaffen und Berechnen von geometrischen Daten verwendet wird, die umfasst:
- einen transparenten Träger (13), der angepasst ist, ein ophthalmisches Objekt zu tragen;
- Mittel (17) zum Beleuchten dieses Trägers von einer Seite des Trägers;
- eine Videokamera (25) an der anderen Seite des Trägers, die zu dem Träger gerichtet ist und angepasst ist, ein Videosignal zu erzeugen, das für mindestens ein Muster repräsentativ ist, das dem auf dem Träger liegenden ophthalmischen Objekt zugeordnet ist; und
- Mittel (27) zum Analysieren und Verarbeiten eines Signals, die am Eingang das von der Kamera erzeugte Videosignal empfangen und die angepasst sind, die zur Herstellung der Linse vorgesehenen geometrischen Daten zu liefern,
wobei das Verfahren **dadurch gekennzeichnet ist, dass**:
(a) auf dem ophthalmischen Objekt (6, 7) unabhängig von den geometrischen Daten ein Prüfmuster (10, 11) gezeichnet wird, wobei dieses Prüfmuster in Bezug auf jede von zwei zueinander senkrechten Achsen asymmetrisch ist;
(b) das ophthalmische Objekt auf dem transparenten Träger (13) der Vorrichtung (12, 112) zum Beschaffen und Anzeigen positioniert wird;
(c) optisch das Prüfmuster mittels der Vorrichtung (12, 112) erfasst und analysiert wird; und
(d) aus der Analyse des Prüfmusters die Seite des ophthalmischen Objekts (6, 7), auf der das auf dem Träger (13) positionierte Objekt ruht, bestimmt wird,
wobei die Vorrichtung (12; 112) für Standardbilder des Prüfmusters (10, 11) repräsentative Informationen für jedes der ophthalmischen Objekte (6, 7) und für jede der möglichen Positionierarten dieser ophthalmischen Objekte enthält und dass der Schritt (c) mittels der Vorrichtung in der folgenden Weise durchgeführt wird:
- (c 1) Korrigieren der Orientierung des Bildes des ophthalmischen Objektes,
- (c 2) optisches Erkennen des erfassten Prüfmusters; und
- (c3) Vergleichen des erfassten Prüfmusters mit den Standardbildern.

2. Verfahren nach Anspruch 1, **dadurch gekennzeichnet, dass** aus der Analyse des Prüfmusters durchzuführende Berechnungen an den geometrischen Daten bestimmt werden.

3. Verfahren nach Anspruch 1 oder 2, **dadurch gekennzeichnet, dass** die Vorrichtung einen einen Bildschirm (31) aufweisenden Monitor (29) aufweist und dass auf dem Bildschirm (31) des Monitors (29) die Gläser des Brillengestells (6, 7) angezeigt werden, wobei jedes Glas in Bezug auf eine dem Brillengestell zugeordnete Achse des Brillengestells (5) neu ausgerichtet wird.

4. Verfahren nach Anspruch 3, **dadurch gekennzeichnet, dass** außerdem auf dem Bildschirm (31) des Monitors (29) eine Warnung als Funktion der Natur des ophthalmischen Objektes (6, 7), der Seite des zu dem Bildschirm gerichteten Objektes und/oder der Orientierung dieses Objektes um eine Achse senkrecht zum Träger angezeigt wird.

5. Verfahren nach einem beliebigen der Ansprüche 1 bis 4, **dadurch gekennzeichnet, dass** bei Schritt (a) auf das Objekt (6, 7) gleichfalls eine Angabe gezeichnet wird, die repräsentativ für eine Eigenschaft des Brillengestells ist, insbesondere das Abmaß des Nasensteges (4) und dass diese Angabe durch optisches Lesen erkannt wird und von der Vorrichtung für die Berechnung der Daten, die für die Herstellung der Linse vorgesehen sind, verwendet wird.

6. Verfahren nach einem beliebigen der vorhergehenden Ansprüche, **dadurch gekennzeichnet, dass** das Prüfmuster chiral ist.

7. Vorrichtung zum Beschaffen und Anzeigen von geometrischen Daten mindestens eines Musters, das einem ophthalmischen Objekt (6, 7), wie einem Präsentationsglas, einer Augenlinse oder einer Schablone zugeordnet ist, für die Herstellung von ophthalmischen Linsen ähnlich dem Objekt oder komplementär dazu, der Art, die umfasst:
- einen transparenten Träger (13), der angepasst ist, ein ophthalmisches Objekt zu tragen;
- Mittel (17) zum Beleuchten dieses Trägers von einer Seite des Trägers;
- eine Videokamera (25) an der anderen Seite des Trägers, die zu dem Träger gerichtet ist und angepasst ist, ein Videosignal zu erzeugen, das für mindestens ein Muster repräsentativ ist, das dem auf dem Träger liegenden ophthalmischen Objekt zugeordnet ist; und
- Mittel (27) zum Analysieren und Verarbeiten eines Signals, die am Eingang das von der Kamera erzeugte Videosignal empfangen und die angepasst sind, die zur Herstellung der Linse vorgesehenen geometrischen Daten zu liefern,
wobei die Vorrichtung **dadurch gekennzeichnet ist, dass** sie umfasst
- Mittel (45) zum optischen Erkennen und Analysieren des realen Bildes eines Prüfmusters (10, 11), das von dem auf dem transparenten Träger (13) positionierten ophthalmischen Objekt (6, 7) getragen wird, unabhängig von den geometrischen Daten, wobei dieses Prüfmuster in Bezug auf jede von zwei zueinander senkrechten Achsen asymmetrisch ist,
- Mittel zum Bestimmen der Seite des ophthalmischen Objektes (6, 7), auf der das auf dem Träger (13) positionierte ophthalmische Objekt ruht, aus der Analyse des realen Bildes des Prüfmusters (10, 11),
- Mittel (41; 141) zum Speichern von Informationen, die für Standardbilder eines Prüfmusters (10, 11), das in Bezug auf jede von zwei zueinander senkrecht stehenden Achsen asymmetrisch ist, repräsentativ sind, wobei diese Standardbilder jedem ophthalmischen Objekt und den unterschiedlichen möglichen Positionierungen dieses ophthalmischen Objektes auf dem Träger mit einer vorbestimmten Positionierung des ophthalmischen Objektes entsprechen;
- Mittel zum Korrigieren der Orientierung des Bildes des auf dem Träger positionierten ophthalmischen Objektes (8, 9); und
- Mittel (47) zum Vergleichen des realen Bildes und der Standardbilder.

8. Vorrichtung nach Anspruch 7, **dadurch gekennzeichnet, dass** sie Mittel zum Bestimmen von an den geometrischen Daten durchzuführenden Berechnungen aus der Analyse des Prüfmusters umfasst.

9. Vorrichtung nach Anspruch 8, **dadurch gekennzeichnet, dass** sie umfasst:
- einen Monitor (29) der einen Bildschirm (31) aufweist und
- Mittel zum Anzeigen der Gläser des Brillengestells (6, 7) auf dem Bildschirm, wobei automatisch die Gläser an ihre jeweilige rechte oder linke Position neu platziert werden und jedes Glas in Bezug auf eine dem Brillengestell zugeordnete Achse des Brillengestell (5) neu ausgerichtet wird.

10. Vorrichtung nach Anspruch 9, **dadurch gekennzeichnet, dass** sie Mittel (49) zum Anzeigen auf dem Bildschirm einer Warnung (58) als Funktion des ophthalmischen Objektes, der zu dem Bildschirm gerichteten Seite dieses Objektes und/oder der Orientierung dieses Objektes um eine Achse senkrecht zum Träger umfasst.

11. Vorrichtung nach Anspruch 10, **dadurch gekennzeichnet, dass** die Anzeigemittel (49) Mittel zum Anzeigen eines Fensters aufweisen, in dem die Natur des Objektes, die Seite und/oder die Orientierung angegeben werden.

12. Vorrichtung nach einem beliebigen der Ansprüche 7 bis 11, **dadurch gekennzeichnet, dass** das Prüfmuster (10, 11) chiral ist.

## Claims

1. A method for acquiring and computing geometrical data of at least one pattern associated with an ophthalmic object (6, 7), such as a presentation lens, an ophthalmic lens or a template for manufacturing ophthalmic lenses similar to the object or complementary thereto, the method being of the type in which a device (12; 112) for acquiring and computing geometrical data is used, said device comprising:
- a transparent support (13) adapted for bearing an ophthalmic object;
- on one side of the support, means (17) for illuminating this support;
- on the other side of the support, a video camera (25) oriented towards the support and adapted for producing a video signal representative of at least one pattern associated with the ophthalmic object laid on the support; and
- signal processing and analysis means (27) receiving as input the video signal produced by the camera, and adapted so as to compute and provide geometrical data intended for making the lens,
the method being **characterized in that**:
(a) a verification pattern (10, 11) independent of said geometrical data is traced on the ophthalmic object (6, 7), this verification pattern being asymmetrical relatively to each of two axes perpendicular to each other;
(b) the ophthalmic object is positioned on the transparent support (13) of the acquisition and display device (12, 112);
(c) by means of said device (12, 112), said verification pattern is optically captured and analyzed; and
(d) from the analysis of the verification pattern, the face of the ophthalmic object (6, 7) on which rests the ophthalmic object (6, 7) positioned on the support (13) is determined,
said device (12; 112) containing information representative of standard images of said verification pattern (10, 11) for each of the ophthalmic objects (6, 7) and for each of the possible positioning modes of these ophthalmic objects, and **in that** step (c) is carried out by means of the device in the following way:
- (c1) correcting the orientation of the image of the ophthalmic object.
- (c2) optically recognizing said captured verification pattern; and
- (c3) comparing the captured verification pattern with said standard images.

2. The method according to claim 1, **characterized in that** from the analysis of said verification pattern, the computations to be carried out on said geometrical data are determined.

3. The method according to claim 1 or 2, **characterized in that** the device includes a monitor (29) including a screen (31), and **in that** the lenses of the frame (6, 7) are displayed on the screen (31) of the monitor (29), by having automatically replaced the lenses in their respective right or left positions, and by having re-oriented each lens relatively to a frame axis (5) associated with the frame.

4. The method according to claim 3, **characterized in that** an alert depending on the nature of the ophthalmic object (6, 7), on the face of the object directed towards the screen and/or on the orientation of this object around an axis perpendicular to the support is further displayed on the screen (31) of the monitor (29).

5. The method according to any of claims 1 to 4, **characterized in that**, in step (a), an indication representative of a characteristic of a frame, notably the measurement of the bridge (4), is also traced on the object (6, 7), and **in that** this indication is recognized by optical read out and is used by said device for computing the data intended for making the lens.

6. The method according to any of the preceding claims, **characterized in that** the verification pattern is chiral.

7. A device for acquiring and displaying geometrical data of at least one pattern associated with an ophthalmic object (6, 7), such as a presentation lens, a lens or a template, for manufacturing ophthalmic lenses similar to the object or complementary thereto, of the type comprising:
- a transparent support (13) adapted for bearing an ophthalmic object;
- on one side of the support, means (17, 45) for illuminating this support;
- on the other side of the support, a video camera (25) oriented towards the support and adapted for producing a video signal representative of at least one pattern associated with the ophthalmic object laid on the support; and
- signal processing and analysis means (27) receiving as input the video signal produced by the camera, and adapted for computing and providing geometrical data intended for making the lens;
the device being **characterized in that** it comprises:
- means (45) for optically recognizing and analyzing the actual image of a verification pattern (10, 11) independently of said geometrical data borne by the ophthalmic object (6, 7) positioned on the transparent support (13), this verification pattern being asymmetrical relatively to each of two axes perpendicular to each other,
- means for determining, from the analysis of the actual image of said verification pattern (10, 11), the face of the ophthalmic object (6, 7) on which rests the ophthalmic object (6, 7) positioned on the support (13)
- means (41; 141) for storing representative information of standard images, for at least one ophthalmic object (6, 7), of a verification pattern (10, 11) asymmetrical relatively to each of two axes perpendicular to each other, these standard images corresponding to each ophthalmic object and to the different possible positionings of this ophthalmic object on the support with a predetermined orientation of the ophthalmic object;
- means for correcting the orientation of the image of the ophthalmic object (8, 9) laid on the support; and
- means (47) for comparing said actual image and said standard images.

8. The device according to claim 7, **characterized in that** it comprises means for determining the computations to be made on said geometrical data from the analysis of said verification pattern.

9. The device according to claim 8, **characterized in that** it comprises:
- a monitor (29) including a screen (31); and
- means for displaying on the screen (31) the lenses of the frame (6,7) by having automatically replaced the lenses in their respective right or left positions, and having reoriented each lens relatively to a frame axis (5) associated with the frame.

10. The device according to claim 9, **characterized in that** it comprises means (49) for displaying on the screen an alert (58) depending on the ophthalmic object, on the face of this object directed towards the screen and/or on the orientation of this object around an axis perpendicular to the support.

11. The device according to claim 10, **characterized in that** said display means (49) include means for displaying a window in which are indicated the nature of the object, of said face and/or of said orientation.

12. The device according to any of claims 7 to 11, **characterized in that** the verification pattern (10, 11) is chiral.
